# EUROPEAN PATENT SPECIFICATION

(11) **EP 3 424 464 B1**
(45) Date of publication and mention of the grant of the patent: **24.06.2020**
(21) Application number: 18182179.4
(22) Date of filing: 06.07.2018
(51) Int. Cl.: A61F 2/16

(54) **INTRAOCULAR LENS SYSTEM FOR IMPLANTATION INTO A LENS CAPSULE OF AN EYE OF HUMAN OR ANIMAL BEING**
INTRAOKULARES LINSENSYSTEM ZUR IMPLANTATION IN EINE LINSENKAPSEL EINES AUGES EINES MENSCHEN ODER TIERS
SYSTÈME DE LENTILLE INTRAOCULAIRE DESTINÉ À ÊTRE IMPLANTÉ DANS LA CAPSULE DU CRISTALLIN D'UN IL D'ÊTRE HUMAIN OU D'ANIMAL

(30) Priority: 07.07.2017 NL 2019196; 20.09.2017 NL 2019584
(43) Date of publication of application: 09.01.2019
(73) Proprietor: Sharpsight B.V., 6049 MG Herten (NL)
(72) Inventor: GONÇALVES, Arnaldo, 6049 MG HERTEN (NL)
(74) Representative: Algemeen Octrooi- en Merkenbureau B.V.

(56) References cited:
- WO-A1-2013/112589
- WO-A1-2016/122805
- WO-A2-2005/013850
- US-A1- 2004 106 993

## Description

### BACKGROUND OF THE INVENTION

This disclosure relates to an intraocular lens system for implantation into a lens capsule of an eye of human or animal being.

The eye of any living being, but in particular of a human or animal being is the sense organ capable of vision. The lens in the eye can be altered in shape resulting in a change of the focal distance of the eye. Through this phenomenon called accommodation the eye is allowed to focus on objects at various distances. Herewith the eye - under normal physical functionality - is able to form a sharp image of the object of interest on the retina.

Due to age or disease the lens can become less transparent or cloudy, and as such the amount of light impinging on the lens will diminish, less light will therefor reach the retina and vision (its sharpness) of the human or animal being will deteriorate. In medial terms, such deficiency in the lens of the eye is categorized as cataract. Treating cataract involves the surgical removal of the lens from the capsular bag and replacing the affected lens with an artificial intraocular lens (IOL) in the capsular bag.

The surgical technique for replacing an affected, cataractous lens with an artificial IOL is called phacoemulsification. This surgical technique can also be used to achieve a refractive goal by patients, that have no cataract at all. The surgical procedure starts with creating a small opening in the anterior side of the capsular bag. Next a thin phacoemulsification-cutting tip is inserted through said opening and into the affected lens. Through ultrasonic vibrations the affected lens is liquefies, allowing the lens to be removed from the capsule bag through aspiration. After removal of the affected, cataractous lens an artificial IOL is placed inside the now empty capsular bag.

After the phacoemulsification treatment it is to be determined whether the replacement of the affected lens with the IOL has provided the desired optical (refractive) correction. Unfortunate in several instances, the treatment may result in an erroneous or less positive correction and that the patient may need correction, such as a stronger refractive correction, an astigmatism correction, or a multifocal correction. Other possibility is that the implanted lens (IOL) can get displacemed inside the lens capsule (subluxation) or it can be lightly positioned outside the desirable optical axis. In certain instances is might be necessary to remove the IOL and replace it with a new IOL. This might tend to additional health issues and discomfort to the patient and in particular to irrevocable damage to the capsular bag, such as posterior rupture.

WO2013/112589 discloses a modular IOL consisting of a base component and a lens component allowing for the removal and replacement of the lens component part in the event of an incorrect optical correction. However this invention is limited by just being able to remove the incorrect lens. This prior art document requires the insertion of the surgical tool into the capsular bag to remove the incorrect lens component and replace it with a new one and is not able to correct the position of the optical lens component into the optical as, when its base seems to be displaced from this desirable position relative to the optical axis. WO 2016/122805 discloses a modular IOL systems including a base and a lens, wherein the lens includes fixed and actuatable tabs for connection to the base.

It is a goal of the present invention to provide a modular IOL allowing correct or modify the final, optical result without the need to remove (any part of) the IOL or manipulate the capsular bag. In particular, it is the goal of the present invention to provide a modular IOL allowing not only the correction of the optical dioptric results by removal of the optical lens, but also to allow the displacement of the optical lens inside the desirable optical axis, despite of the position of the base component. This invention also provides the option of providing a modular IOL allowing to correct or to modify the final, optical result without the need to remove (any part of) the IOL inside the capsular bag which makes it easier to manipulate and with less risk of damage of the capsular bag.

### SUMMARY OF THE DISCLOSURE

In a first aspect, an intraocular lens system for implantation into a lens capsule of an eye of human or animal body is presented, said lens capsule having a capsulorhexis perimeter, said intraocular lens system comprising:
i) an intraocular primary component having a body exhibiting a flat configuration; and
ii) an intraocular secondary component having an optical body with an optical axis,
wherein the primary component includes a first primary component body part configured to fit within the lens capsule and a second primary component body part configured to be positioned outside the lens capsule and in the posterior chamber of the eye and wherein the second primary component body part is configured to releasably receive the secondary component in the posterior chamber of the eye to provide optical correction, such that the secondary component is movable relative to the primary component.

Herewith it is possible after implanting the modular IOL to adapt the orientation of the secondary component within the capsular bag and as such to adapt the optical performance of the modular IOL without the need of removal and replacement nor of manipulation of the capsular bag. Also the location of a part of the first primary component within the lens capsule provides additional support to the lens capsule.

In another example the second primary component body part is configured to be positioned at the same level as the anterior surface line of the lens capsule and in the posterior chamber of the eye.

In a further example the primary component includes a center hole extending in an anterior-posterior direction, wherein the hole is sized and configured to receive the secondary component. This allows for an easy access and correction of the secondary, lens component relative to the primary, base component.

In an example having limited dimensions seen in the direction of the optical axis of the eye the optical body of the secondary component is accommodated within the plane formed by the flat configuration of the second body part of the primary component.

In another example the optical body of the secondary component is positioned outside the plane formed by the flat configuration of the second body part of the primary component. Albeit its enlarged dimension seen in the direction of the optical axis of the eye still embodiment allows for easier access and manipulation of the secondary component for optical correction.

In an example which guarantees a stable and fixed positioning of the primary component in the capsular bag, the primary component is provided with two or more primary support flanges extending from the first body part. The support flanges extends and abut against inner circumference of the capsular bag and thus provides stability to the base component and allow a more efficient correction of the secondary, optical component relative to the stabilized primary, base component.

In particular said primary support flanges extend in the plane formed by the flat configuration of the first body part of the primary component, thus maintaining a limited dimension seen in the direction of the optical axis of the eye.

In yet another example said primary support flanges extend outside the plane formed by the flat configuration of the first body part of the primary component, thus positioning the primary base component and as such also the secondary, optical component in the anterior direction improving access and manipulation of the latter.

Furthermore in an example the secondary component comprises multiple secondary flanges extending away from the optical axis and in the plane formed by the optical body of the secondary component, wherein each secondary flange is movable accommodated in an flange opening provided in the circumference of the second body part of the primary component. This guarantees a movable yet enclosed secondary, optical component relative to the fixed primary, base component.

In an example each secondary flange is provided with an opening, which serves as the contact point for a tip or pin shaped surgical tool for repositioning the secondary component relative to the primary component.

More in particular each secondary flange is provided with a rounded edge, said rounded edge arranged for hooking around the outer circumference of first primary component body part. This allows for a secure mounting of the secondary component to the primary component.

In yet another example the primary component constitutes a support and the secondary component constitutes a lens.

In yet another embodiment the primary component is manufactured from a material having a contrast enhancing color relative to the secondary, optical component. Such contrast enhancements e.g. by using color differences it is possible to the optical system to enhance a better contrast resolution and a better visual acuity as well as reducing possible complains of disfothopsy due to restricting light dispersion through the periphery of the optic system.

### BRIEF DESCRIPTION OF THE DRAWINGS

The accompanying drawings facilitate an understanding of the various embodiments:
Figure 1 a cross section of an eye of a human being;
Figure 2 is an example of an intraocular lens system for implantation into a lens capsule of an eye;
Figure 3 is a cross section of the example of Figure 2;
Figure 4 is another example intraocular lens system for implantation into a lens capsule of an eye;
Figure 5 is a cross section of the example of Figure 4;
Figure 6 yet another example of an embodiment of an intraocular lens system;
Figure 7 the embodiment of Figure 6 in an assembled state;
Figure 8 a detailed view of the embodiment of Figure 6 in the assembled state of Figure 7;
Figure 9 yet another example of an embodiment of an intraocular lens system;
Figure 10 the embodiment of Figure 9 in an assembled state;
Figure 11 a detailed view of the embodiment of Figure 9 in the assembled state of Figure 10.

### DETAILED DESCRIPTION

For a better understanding of the invention like parts in the drawings are to be denoted with like reference numerals.

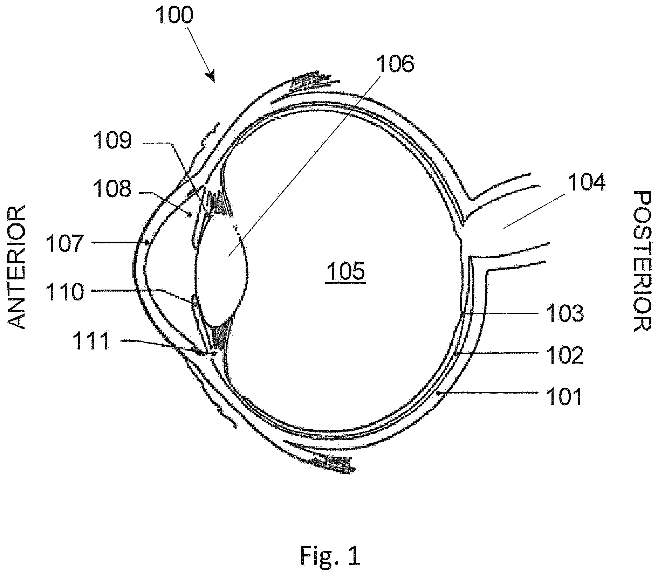

Figure 1 depicts a cross section of an eye, in particular a cross section of the eye 100 of a human being. The eye 100 is a sense organ capable of vision having an almost spherical configuration. Seen in the direction of light impinging on the eye, the anterior, frontal side of the eye 100 is formed by the cornea 107 having a stronger curvature comparted to the posterior side, being the sclera 101. The limbus connects the cornea 107 and the sclera 101. The cornea 107, the iris 110 and the lens capsule 106 form the anterior chamber 108 filled with aqueous humor liquid. Behind the lens 106 the vitreous body 105 is filled with vitreous gel.

The lens 106 is suspended in the eye by means of the ciliary body 111 composed of ciliary muscle and fibers. Reference numeral 109 denoted the posterior chamber present between the lens 106, the ciliary body 111 and the iris 110. The fundus or area opposite to the lens 106 at the posterior side of the eye 100 includes the macula 103, as well as the retina, being the innermost tissue layer containing the rod and cone cells. Also shown is the optic nerve 104 leaving the eye 100 at its posterior side towards the brains. Reference numeral 102 denotes the choroid.

The clear lens 106 is a transparent, biconvex structure in the eye that, along with the cornea 107 refract impinging light to be focused on the retina. The shape of the lens 106 can be changed resulting in a change of the focal distance of the eye 100 allowing the eye to focus (accommodation) on objects at various distances. Herewith the eye - under normal physical functionality - is able to form a sharp image of the object of interest on the retina.

The lens 106 is composed of three parts being the lens capsule, the lens epithelium, and the lens fibers. The lens capsule forms the outermost layer of the lens and the lens fibers form the bulk of the interior of the lens. The cells of the lens epithelium, located between the lens capsule and the outermost layer of lens fibers, are found predominantly on the anterior side of the lens but extend posteriorly just beyond the equator.

The lens capsule is a smooth, transparent basement membrane that completely surrounds the lens. The lens capsule is elastic and is composed of collagen and its shape can be changed for focusing purposes, also called accommodation of the lens 106.

Due to disease or disorder the eye 100 can undergo a surgical treatment in order to replace the affected lens 106 with a modular intraocular lens assembly (IOL) according to the invention. For example, a modular IOL according to one or more embodiments of the present invention can be implemented to treat cataracts, large optical errors in myopic (near-sighted), hyperopic (far-sighted), and astigmatic eyes, ectopia lentis, aphakia, pseudophakia, and nuclear sclerosis.

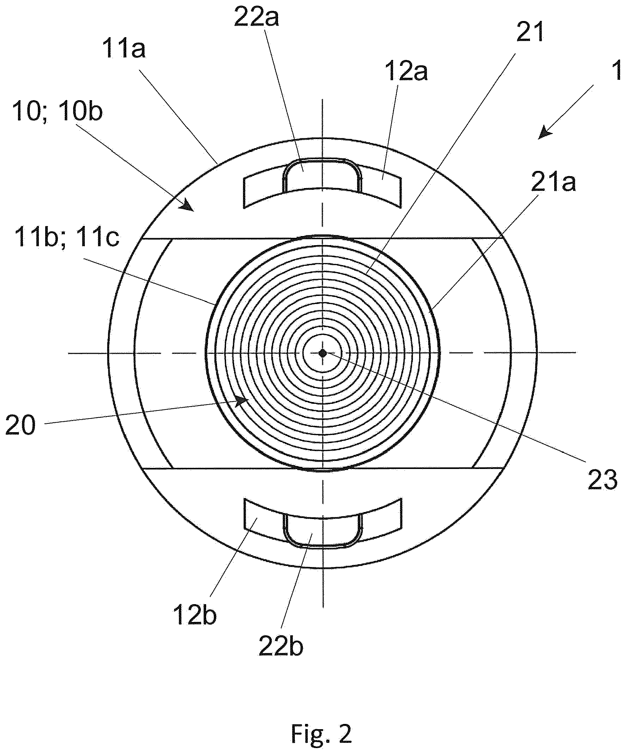

Figure 2 discloses a first embodiment of a modular intraocular lens system (IOL) according to an example of the invention. The IOL is indicated with reference numeral 1 and is composed of a primary component 10 which serves as a base component for implant in the capsular bag of an eye as described in combination with Figure 1 above. The intraocular primary, base component 10 interacts with an intraocular secondary, optical component indicated with reference numeral 20.

The intraocular primary, base component comprises a body 10 exhibiting a flat configuration and comprises a first primary component body part indicated with reference numeral 10a and a second primary component body part indicated with reference numeral 10b. The first primary component body part 10a of the intraocular primary component body 10 is configured to fit within the lens capsule or capsular bag 106 of the eye 100. Preferably, the first primary component body part 10a of the body 10 has an outer dimension or equatorial perimeter which is equal to the inner perimeter of the capsular bag or lens capsule 106.

The second primary component body part 10b of the intraocular primary component body 10 is configured to be positioned outside the lens capsule or capsular bag 106 and in the posterior chamber 109 of the eye. In a preferred, yet alternative embodiment the second primary component body part is configured to be positioned at the same level as the anterior surface line of the lens capsule and in the posterior chamber of the eye. The outer perimeter or outer circumference of the second primary component body part 10b of the intraocular primary, base component 10 is indicated with reference numeral 11a. In another example the first primary component body part 10a of the primary, base component 10 may have an equatorial perimeter greater than or equal or even smaller to the inner, capsulorhexis perimeter of the capsular bag 106.

The intraocular secondary, optical component 20 consists of an optical body 21 with an optical axis 23 which also is the cylindrical symmetric axis of the complete assembly of the intraocular lens system (primary and secondary components assembled together). In an example the outer dimensions of the intraocular secondary, optical component 20 are less than the outer dimensions of the second primary component body part 10b of the intraocular primary, base component 10. This allows for and guarantees a proper support and mounting of the secondary component 20 on (and/or in) the second primary component body part 10b.

In another example the secondary component may have an outer dimension , which is equal or smaller than than the capsulorhexis perimeter.

The primary, base component 10 and the secondary, optical component 20 of the modular IOL system 1 may be formed of the same, similar or different materials. Suitable materials may include, for example, acrylate, base materials, silicon materials, hydrophobic polymers or hydrophilic polymers. Such materials may have shape-memory characteristics. For example, materials comprising the optical parts of the IOL system can be silicon, PMMA, hydrogels, hydrophobic acrylic, hydrophilic acrylic and/or other transparent materials commonly used for intraocular lenses. The non-optical parts of the modular IOL assembly might include nitinol, polyethylene sulfone and/or polyimide.

In general it should be noted that the modular IOL system according to the invention is composed of two components, indicated already as the intraocular primary (base) component 10 (comprising the interconnected first and second primary body parts 10a and 10b) and intraocular secondary (optical) component 20. It is noted that the intraocular primary, base component functions as a support base for accommodating the intraocular secondary, optical component. As such, but not limited, the primary, base component 10 only performs a support function and does not provide any optical correction, the latter function is solely or predominantly performed by the intraocular secondary, optical component 20.

However, in yet another example it is also possible that the primary, base component 10 does provide some optical correction together with the secondary, optical component 20 and as such the modular IOL system is composed of a primary lens and a secondary lens part.

The positioning or implanting of the modular IOL system 1 in the capsular bag of the eye 100 is performed by a suitable, known ophthalmology surgical tools, in which the modular IOL is accommodated in a folded or rolled-up manner. Once inserted in the capsular bag, the rolled-up modular IOL system is to be ejected from or driven out the surgical tool into the capsular bag and during the ejection or insertion in the capsular bag the rolled-up modular IOL system automatically unrolls into its flat configuration as depicted in Figure 2. In the unrolled configuration the modular IOL system occupies the space in the capsular bag.

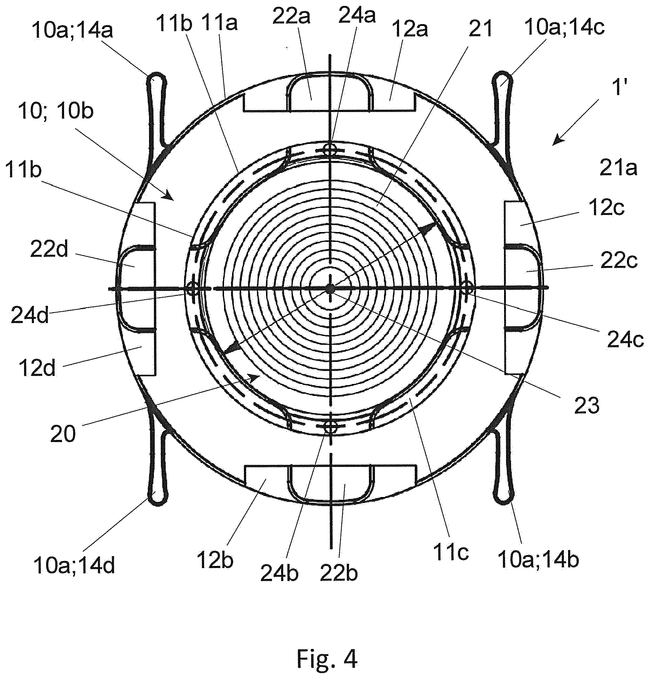

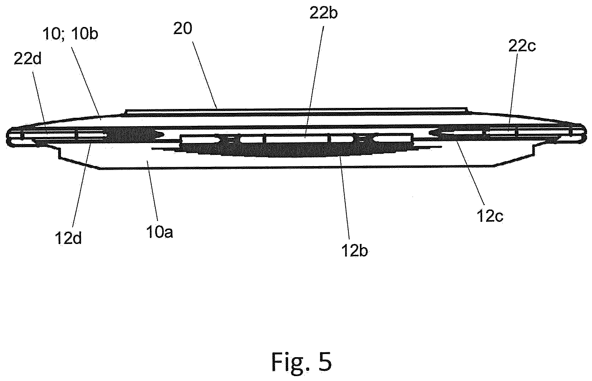

The second primary component body part 10b of the primary, base component 10 is provided with a center hole 11c defining an inner circumference 11b. Within said center hole 11c the secondary, optical component 20, and in particular the optical body 21 of the secondary, optical component 20, can be accommodated. As such, the outer circumference 21a of the optical body 21 of the secondary, optical component 20 is more or less conformal to the inner circumference 11b of the center hole 11c provided in the second primary component body part 10b of the primary, base component 10. In another embodiment, the outer circumference 21a of the optical body 21 of the secondary, optical component 20 is slightly smaller than the inner circumference 11b of the center hole 11c. This embodiment is depicted in Figure 4 and Figure 5.

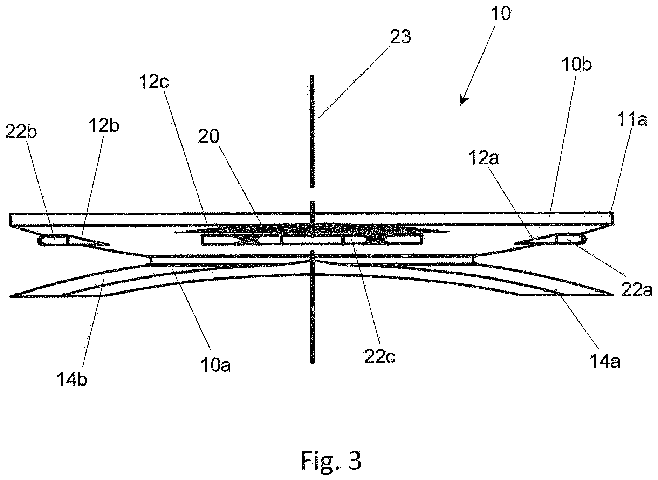

The accommodation of the optical body 21 of the secondary, base component 20 in the center hole 11c of the second primary component body part 10b of the primary, base component 10 allows for an adjustment of the optical body 21 (hence the optical axis 23 thereof) relative to the primary, base component 10. As such, with the modular IOL according to the examples as shown in Figure 2 (and Figure 3) as well as in Figure 4 (and Figure 5) it is possible to correct the optical dioptric characteristic of the modular IOL. This is performed by replacement of the secondary, optical component 20 (the optical lens) with another example of a secondary, optical component without the removal of the complete IOL from the capsular bag. This is beneficial for the welfare of the patient during treatment, as the surgical treatment is less complex and burdensome, as the surgical removal and replacement of the secondary, optical component 20 takes place in the posterior chamber 109 of the eye. This, because the first primary component body part 10a of the primary, base component 10 remains inside the capsular bag or lens capsule 106, whereas the second primary component body part 10b of the primary, base component 10 containing the secondary, optical component 20 to be replaced is positioned in the posterior chamber 109 and thus easy access and manipulation by the surgeon is allowed whilst replacing the secondary, optical component 20. Herewith, the surgical procedure is less drastic compared to the implanting/replacement techniques associated with prior art modular IOLs.

In addition, the modular IOL according to the examples of the present invention also allows for an easy manipulation, displacement or even removal of the secondary, optical component accommodated in the second primary component body part 10b positioned in the posterior chamber 109 relative to the first primary component body part 10a the primary, base component 10 being deployed in the capsular bag 106. In particular, the optical body 21 can be displaced relative to the primary, base component 10 by rotating around the optical axis 23 or, as shown in the example of Figure 4 and Figure 5, by displacing the optical body 21 in a transverse direction over a certain distance relative to the optical axis 23 within the oversized center hole 11c provided in the second primary component body part 10b of the primary, base component 10.

In either embodiment as disclosed in Figure 2 and Figure 4, the secondary, optical component 20 is provided with several secondary flanges, two flanges in Figure 2 indicated with reference numeral 22a-22b (in Figure 2 and Figure 3) and four flanges indicated with reference numerals 22a-22d in Figures 4 and 5. The secondary flanges 22a-22d are accommodated in suitable flange openings 12a-12d provided in the second primary component body part 10b of the primary, base component 10.

Herewith, a proper confinement of the secondary, optical component 20 in the second primary component body part 10b of the primary, base component 10 is guaranteed, as the first primary component body part 10a of the primary, base component 10 is properly and securely placed inside the capsular bag 106 and thus forms a stable support face for the secondary component 20.

Proper confinement or retaining of the first primary component body part 10a of the primary, base component 10 within the capsular bag 106 is guaranteed as in a further example the first primary component body part 10b of the primary, base component 10 is provided with several support flanges 14a-14d. In one embodiment, the support flanges 14a-14d extend from the first primary component body part 10a of the primary, base component 10. In particular, as shown in the embodiment of Figure 2 and in particular Figure 3, the support flanges 14a-14d extend outside and away from the plane formed by the flat configuration of the first primary component body part 10a of the primary, base component 10.

As such, the second primary component body part 10b of the primary, base component 10 together with the secondary, optical component 20 contained therein are positioned further in the anterior direction which improves access and manipulation of the secondary, optical component 20 in order to adjust or improve its orientation relative to the optical axis 23 and/or the primary, base component 10.

In another example, the support flanges 14a-14d extend away from the outer circumference of the first primary component body part 10a of the primary, base component 10, whereas the support flanges 14a-14d are also positioned and extending within the plane formed by the first primary component body part 10a of the primary, base component 10. This latter configuration is shown in Figures 4 and 5 resulting in a modular IOL having a limited dimension seen in the direction of the optical axis of the eye (anterior-posterior direction).

In either embodiment the support flanges 14a-14d extending from the first primary component body part 10a of the primary, base component 10 abut against the inner circumference of the capsular bag 106 and as such provide a stability to the primary, base component and also allow a more efficient correction of the secondary, optical component 20 relative to the stabilized primary, base component 10.

Correcting the position of the secondary, optical component 20 relative to the second primary component body part 10b of the primary, base component 10 is further effectuated due to the presence of adjustment openings 24a-24d in every secondary flange 22a-22d. The adjustment openings 24a-24d serve as contact or gripping point for the tip of a surgical tool such as sinskey hooks for displacing the secondary, optical component 20 accommodated inside the center hole 11c of the second primary component body part 10b of the primary, base component 10 relative to the optical axis 23. As each secondary flange 22a-22d is accommodated in a flange opening 12a-12d of the second primary component body part 10b of the primary, base component 10, manipulation of the secondary, base component 20 using the tip of a surgical tool positioned in one of the adjustment or gripping openings 24a-24d may result in a limited rotation of the optical body 20 around the optical axis 23. Also a (slight) transverse displacement of the optical body 20 relative to the optical axis 23 can herewith be effected, for example in either X- or Y-direction wherein the X- and Y-directions denote orthogonal axes both intersecting the optical axis 23 and running across the opposite secondary flanges 22a-22b and 22c-22d respectively.

In particular, the adjustment or gripping openings 24a-24b are positioned exactly opposite to each other on an equatorial or diametrical line crossing the optical axis 23. The same applies to the adjustment or gripping openings 24c and 24d which are also located opposite to each other on an equatorial (diametrical) line crossing the optical axis 23 and located perpendicular to the equatorial (diametrical) line between the adjustment openings 24a-24b and the optical axis 23.

The adjustment of the optical body 20 relative to the second primary component body part 10b of the primary, base component 10 by means of rotation around the optical axis 23 or transverse displacement of the optical body 20 within the oversized center hole 11c as shown in the example of Figure 4, allows for a proper correction of the optical axis 23 relative to the macula and retina 103 of the eye.

The embodiments as shown in Figures 2 and 3 as well as in Figures 4 and 5 allow for a less drastic replacement of the secondary, optical component 20 contained in the second primary component body part 10b from the static first primary component body part 10a of the primary, base component 10 which first body part 10a remains in the capsular bag 106, as outlined before. The examples also allow for correction of the orientation of the secondary, optical component 20 relative to the macula and retina of the eye.

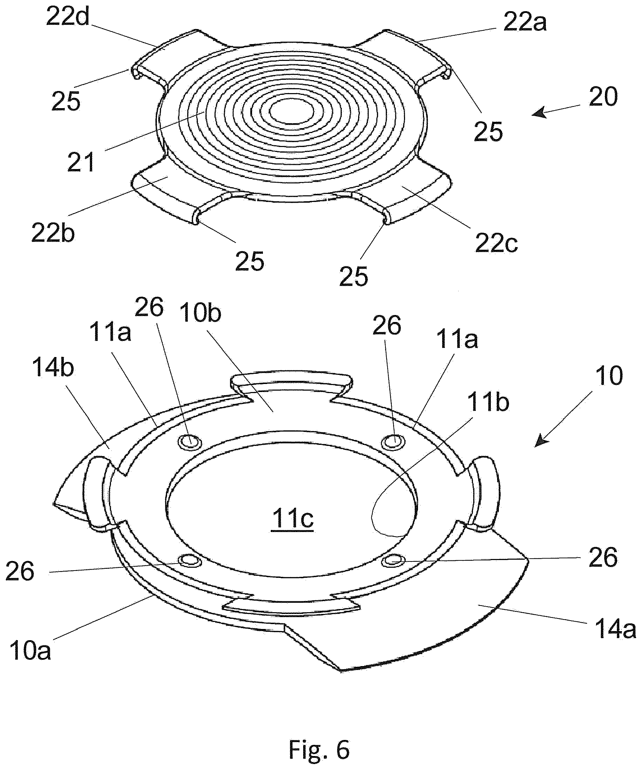

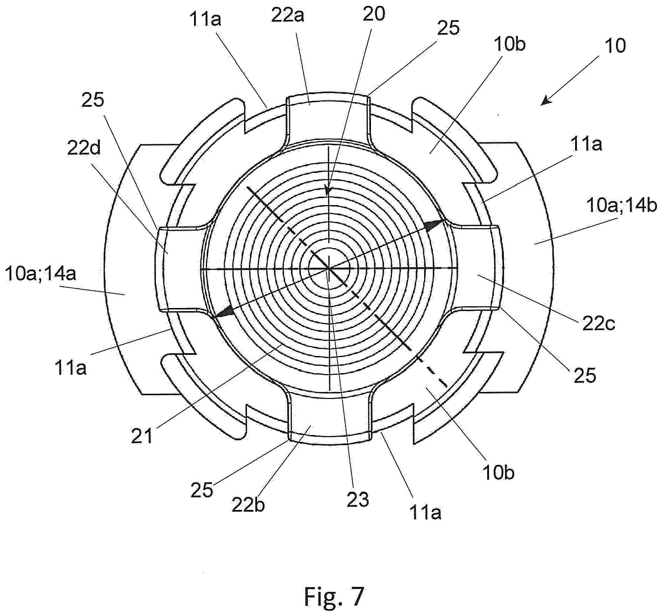

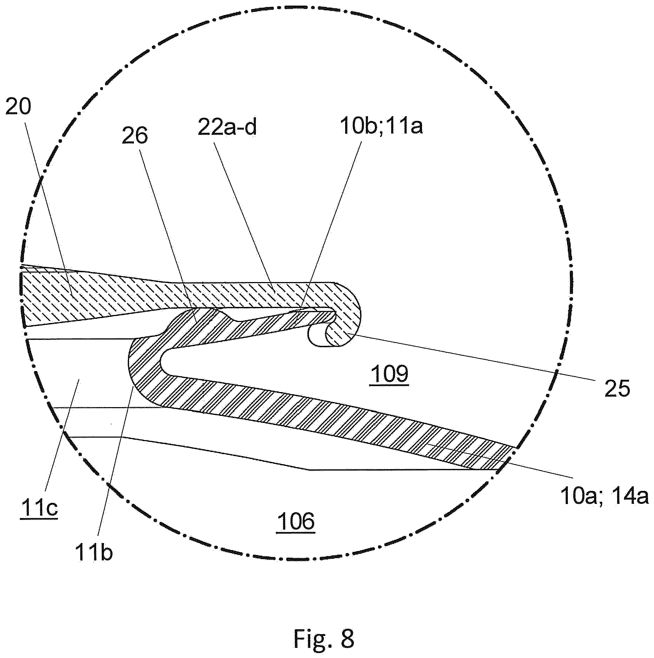

In Figure 6 yet another example of an embodiment of an intraocular lens system according to the invention is shown, combined with Figure 7 showing the embodiment of Figure 6 in an assembled state and Figure 8 showing a detailed view of the embodiment of Figure 6 in the assembled state of Figure 7. Also in the Figures 6-8 like parts are denoted with like reference numerals as used in the Figures 1-5.

Exhibiting a similar functionality as the embodiments discussed above, the modular IOL system as shown in Figures 6-8 is also composed of two components, reference numerals 10 and 20. The intraocular primary, base component comprises a body 10 exhibiting a flat configuration and comprises a first primary component body part indicated with reference numeral 10a and a second primary component body part indicated with reference numeral 10b. The first primary component body part 10a of the intraocular primary component body 10 is configured to fit within the lens capsule or capsular bag 106 of the eye 100. Preferably, the first primary component body part 10a of the body 10 has an outer dimension or equatorial perimeter which is equal to the inner perimeter of the capsular bag or lens capsule 106.

The second primary component body part 10b of the intraocular primary component body 10 is configured to be positioned outside the lens capsule or capsular bag 106 and in the posterior chamber 109 of the eye. The outer perimeter or outer circumference of the second primary component body part 10b of the intraocular primary, base component 10 is indicated with reference numeral 11a.

Similarly, the intraocular secondary, optical component 20 consists of an optical body 21 having an optical axis 23, which also is the cylindrical symmetric axis of the complete assembly of the intraocular lens system (primary and secondary components assembled together). In this embodiment the secondary, optical component 20 is provided with several secondary flanges at its the outer circumference 21a, in particular four flanges indicated with reference numerals 22a-22d (similar as in Figures 4 and 5). However also two flanges 22a-22b can be implemented similar as the embodiment in Figure 2 and 3, or even three flanges. In all flange configurations having 2, 3 or 4 flanges, it is preferred to have the flanges positioned equidistant at the outer circumference 21a of optical body of secondary, optical component 20.

This facilitates the placement of the secondary, optical component 20 on the second primary component body part 10b of the intraocular primary component body 10 and allows also for easy adjustment by rotating of the optical body 21 relative to the primary, base component 10 around the optical axis 23.

In this embodiment each flange 22a-22d is provided with a rounded edge 25, a detail of the rounded edge 25 being shown in Figure 8. Said rounded edge 25 is arranged for hooking around the outer circumference edge 11a of first primary component body part 10. The hooking functionality is effectuated by a slight deformation of each flange 22a-22d due to its flexible material by a bending force and hooking the rounded edge 25 around the outer circumference edge 11a as shown in Figure 8, allowing the rounded edge 25 to snap or click behind the edge 11a and creating an interlocking connection between the secondary, optical component 20 on the second primary component body part 10b of the intraocular primary component body 10.

This interlocking connection can easily be released or removed by applying a proper deformation force on each flange 22a-22d, thereby unhooking the rounded edge 25 from the circumference edge 11a for example for replacement of the secondary, optical component 20.

This interlocking connection also allows for an easy adjustment of the secondary, optical component 20 by rotating of the optical body 21 relative to the primary, base component 10 around the optical axis 23. The adjustment of the secondary, optical component 20 by rotating relative to the second primary component body part 10b of the primary, base component 10 can be facilitated as additionally the outer circumference edge 11a is provided with one or more support knobs 26. These knobs 26 support the secondary, optical component 20 on several distinct supporting points thereby reducing the overall contact surface between the secondary, optical component 20 and the second primary component body part 10b, thus reducing friction during adjustments.

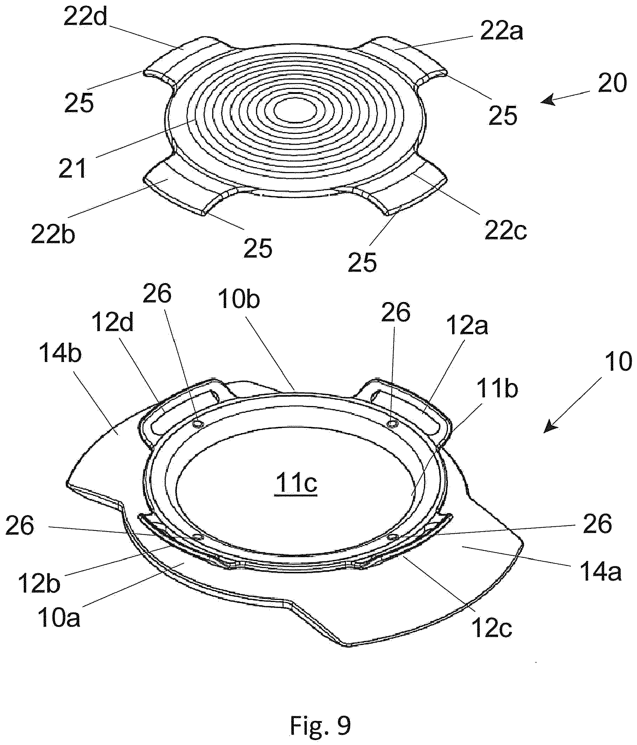

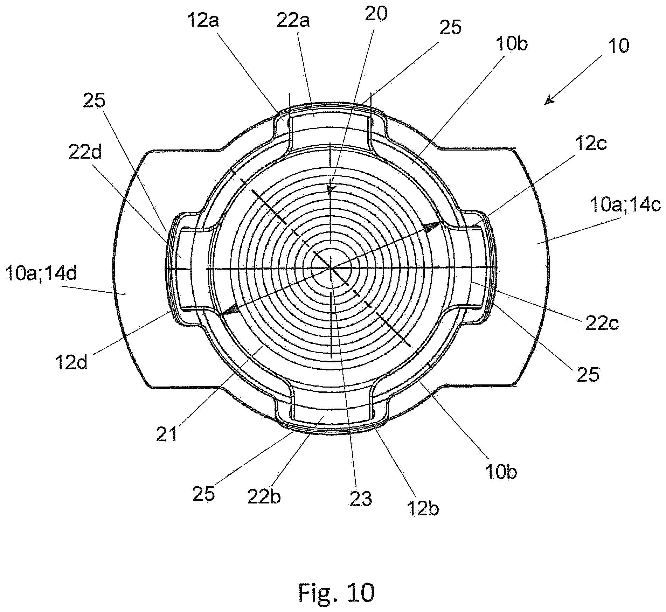

Another embodiment as shown in the Figures 9-10-11 shows significant resemblance with the embodiment of Figure 4. Here the modular IOL system as shown is also composed of two components, reference numerals 10 and 20. The intraocular primary, base component comprises a body 10 exhibiting a flat configuration and comprises a first primary component body part indicated with reference numeral 10a and a second primary component body part indicated with reference numeral 10b. The first primary component body part 10a of the intraocular primary component body 10 is configured to fit within the lens capsule or capsular bag 106 of the eye 100. Preferably, the first primary component body part 10a of the body 10 has an outer dimension or equatorial perimeter which is equal to the inner perimeter of the capsular bag or lens capsule 106. The second primary component body part 10b of the intraocular primary component body 10 is configured to be positioned outside the lens capsule or capsular bag 106 and in the posterior chamber 109 of the eye.

The secondary, optical component 20 is provided with several (four) flanges indicated with reference numerals 22a-22d. The secondary flanges 22a-22d are accommodated in suitable flange openings 12a-12d provided in the second primary component body part 10b of the primary, base component 10. Herewith, a proper confinement of the secondary, optical component 20 in the second primary component body part 10b of the primary, base component 10 is guaranteed, as the first primary component body part 10a of the primary, base component 10 is properly and securely placed inside the capsular bag 106 and thus forms a stable support face for the secondary component 20.

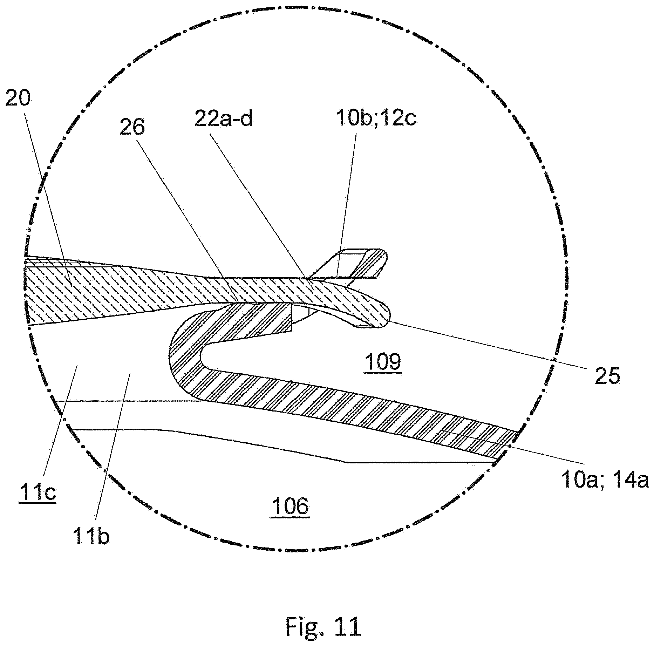

Proper confinement or retaining of the first primary component body part 10a of the primary, base component 10 within the capsular bag 106 is guaranteed as in a further example the first primary component body part 10b of the primary, base component 10 is provided with several support flanges 14a-14b. In this embodiment, the support flanges 14a-14b extend from the first primary component body part 10a of the primary, base component 10. In particular, as shown in the embodiment of Figure 9 and in particular Figure 11, the support flanges 14a-14b extend outside and away from the plane formed by the flat configuration of the first primary component body part 10a of the primary, base component 10.

As such, the second primary component body part 10b of the primary, base component 10 together with the secondary, optical component 20 contained therein are positioned further in the anterior direction which improves access and manipulation of the secondary, optical component 20 in order to adjust or improve its orientation relative to the optical axis 23 and/or the primary, base component 10.

The support flanges 14a-14b extending from the first primary component body part 10a of the primary, base component 10 abut against the inner circumference of the capsular bag 106 and as such provide a stability to the primary, base component and also allow a more efficient correction of the secondary, optical component 20 relative to the stabilized primary, base component 10.

In all embodiments the first primary component body part of the primary component may provide additional support to the lens capsule, whereas alternatively the second primary component body part is configured to be positioned at the same level as the anterior surface line of the lens capsule and in the posterior chamber of the eye. Additionally the contact between the optic system and the posterior surface of the lenticular capsule is prevented and thus the formation of posterior capsular opacity is avoided.

The static primary, base component 10, which is to receive the secondary, optical component 20 in the second primary component body part 10b can be chosen to be manufactured from a material having contrast enhancing color. Such a color contrasts with the color of the material of the secondary, optical component 20. For example, the color of the material of the static primary, base component 10 can be darker that the color of the material of the secondary, optical component 20. The static primary, base component 10 can also be provided locally with a contrast enhancing color.

For example, certain parts of the static primary, base component 10 can have a color difference with respect to the color of the material of the secondary, optical component 20 as well as with respect to other parts of the static primary, base component 10 at locations on the static primary, base component 10 closely or near the secondary, optical component 20 being accommodated in the second primary component body part 10b of the primary, base component 10. In an example, said color difference can be applied to the circumference 11b of the center hole 13, thereby enhancing the contrast between the secondary, optical component 20 being accommodated in the center hole 13.

With the above contrast enhancements (color differences) it is possible to the optical system to enhance a better contrast resolution and a better visual acuity as well as reducing possible complains of disfothopsy due to restricting light dispersion through the periphery of the optic system. Moreover, as it can function as a partial closed esfincter, this also avoids the creation of glare at night.

### LIST OF REFERENCE NUMERALS

- 1: first embodiment of modular intraocular lens system
- 1': second embodiment of modular intraocular lens system
- 10: primary, base component
- 10a: first primary component body part
- 10b: second primary component body part
- 11a: outer circumference of second primary component body part
- 11b: inner circumference of center hole of second primary component body part
- 11c: center hole of primary, base component 10
- 12a-12d: flange opening of second primary component body part
- 13: center hole in first primary component body part
- 14a-14d: primary component support flanges
- 20: secondary, optical component
- 21: optical body of secondary, optical component
- 21a: outer circumference of optical body of secondary, optical component
- 22a-22d: secondary flanges of secondary, optical component
- 23: optical axis
- 24a-24d: adjustment opening of secondary flanges
- 25: rounded edge of secondary flange
- 26: support knob on second primary component body part
- 100: eye
- 101: sclera
- 102: choroid
- 103: macula and retina
- 104: optic nerve
- 105: vitrous body
- 106: lens capsule (capsular bag)
- 107: cornea
- 108: anterior chamber
- 109: posterior chamber
- 110: iris
- 111: ciliary body

## Claims

1. An intraocular lens system (1, 1') for implantation into a lens capsule (106) of an eye (100) of human or animal body, said lens capsule (106) having a capsulorhexis perimeter, said intraocular lens system (1,1') comprising:
i) an intraocular primary component (10) having a body exhibiting a flat configuration; and
ii) an intraocular secondary component (20) having an optical body with an optical axis (23),
wherein the primary component (10) includes a first primary component body part (10a) configured to fit within the lens capsule (106) and a second primary component body part (10b) configured to be positioned outside the lens capsule (106) and in the posterior chamber (109) of the eye (100) and wherein the second primary component body part (10b) is configured to releasably receive the secondary component (20) in the posterior chamber (109) of the eye (100) to provide optical correction, such that the secondary component (20) is movable relative to the primary component (10).

2. A system (1) according claim 1, wherein the second primary component body part (10b) is configured to be positioned at the same level as the anterior surface line of the lens capsule (106) and in the posterior chamber (109) of the eye (100).

3. A system (1) according to claim 1 or 2, wherein the primary component (10) includes a center hole extending in an anterior-posterior direction, wherein the hole is sized and configured to receive the secondary component (20) in the second primary component body part. (10)

4. A system (1) according to any one or more of the claims 1-3, wherein the optical body of the secondary component (20) is accommodated within the plane formed by the flat configuration of the second body part (10b) of the primary component (10).

5. A system (1) according to any one or more of the claims 1-3, wherein the optical body of the secondary component (20) is positioned outside the plane formed by the flat configuration of the second body part (10b) of the primary component (10).

6. A system (1) according to any one or more of the preceding claims, wherein the primary component (10) is provided with two or more primary support flanges (14a-14d) extending from the first body part (10).

7. A system (1) according to claim 6, wherein said primary support flanges (14a-14d) extend in the plane formed by the flat configuration of the first body part (10a) of the primary component (10b).

8. A system (1) according to claim 6, wherein said primary support flanges (14a-14d) extend outside the plane formed by the flat configuration of the first body part (10a) of the primary component (10).

9. A system (1) according to any one or more of the preceding claims, wherein the secondary component (20) comprises multiple secondary flanges (22a-22d) extending away from the optical axis (23) and in the plane formed by the optical body of the secondary component (20).

10. A system (1) according to claim 9, wherein each secondary flange (22a-22d) is movable accommodated in an flange opening (12a-12d) provided in the circumference of the second body part (10b) of the primary component (10).

11. A system (1) according to claim 9 or 10, wherein each secondary flange (22a-22d) is provided with an opening (24a-24d).

12. A system (1) according to claim 9, wherein each secondary flange (22a-22d) is provided with a rounded edge (25), said rounded edge (25) arranged for hooking around the outer circumference of first primary component body part (10a).

13. A system (1) according to any one or more of the preceding claims, wherein the primary component (10) constitutes a support and the secondary component (20) constitutes a lens.

14. A system (1) according to any one or more of the preceding claims, wherein the primary component (10) is manufactured from a material having a contrast enhancing color relative to the secondary, optical component (20).

## Patentansprüche

1. Intraokulares Linsensystem (1, 1') zur Implantation in eine Linsenkapsel (106) eines Auges (100) eines menschlichen oder tierischen Körpers, wobei die Linsenkapsel (106) einen Kapsulorhexisumfang aufweist, wobei das intraokulare Linsensystem (1, 1') umfasst:
i) eine intraokulare Primärkomponente (10) mit einem Körper, der eine flache Konfiguration zeigt; und
ii) eine intraokulare Sekundärkomponente (20), die einen optischen Körper mit einer optischen Achse (23) aufweist,
wobei die Primärkomponente (10) ein erstes Primärkomponentenkörperteil (10a), das ausgestaltet ist, um in die Linsenkapsel (106) zu passen, und ein zweites Primärkomponentenkörperteil (10b) einschließt, das ausgestaltet ist, um außerhalb der Linsenkapsel (106) und in der posterioren Kammer (109) des Auges (100) positioniert zu werden, und wobei das zweite Primärkomponentenkörperteil (10b) ausgestaltet ist, um die Sekundärkomponente (20) in der posterioren Kammer (109) des Auges (100) ablösbar zu empfangen, so dass die Sekundärkomponente (20) relativ zu der Primärkomponente (10) beweglich ist.

2. System (1) nach Anspruch 1, wobei das zweite Primärkomponentenkörperteil (10b) ausgestaltet ist, um auf derselben Ebene wie die anteriore Oberflächenlinie der Linsenkapsel (106) und in der posterioren Kammer (109) des Auges (100) positioniert zu werden.

3. System (1) nach Anspruch 1 oder 2, wobei die Primärkomponente (10) ein Mittelloch einschließt, das sich in anterior-posteriorer Richtung erstreckt, wobei das Loch so bemessen und ausgestaltet ist, dass es die Sekundärkomponente (20) in dem zweiten Primärkomponentenkörper (10) aufnehmen kann.

4. System (1) nach einem oder mehreren der Ansprüche 1 bis 3, wobei der optische Körper der Sekundärkomponente (20) innerhalb der Ebene untergebracht ist, die durch die flache Konfiguration des zweiten Körperteils (10b) der Primärkomponente (10) gebildet wird.

5. System (1) nach einem oder mehreren der Ansprüche 1 bis 3, wobei der optische Körper der Sekundärkomponente (20) außerhalb der Ebene positioniert ist, die durch die flache Konfiguration des zweiten Körperteils (10b) der Primärkomponente (10) gebildet wird.

6. System (1) nach einem der vorhergehenden Ansprüche, wobei die Primärkomponente (10) mit zwei oder mehr Primärträgerflanschen (14a-14d) ausgestattet ist, die sich von dem ersten Körperteil (10) erstrecken.

7. System (1) nach Anspruch 6, wobei die Primärträgerflansche (14a-14d) sich in der Ebene erstrecken, die durch die flache Konfiguration des ersten Körperteils (10a) der Primärkomponente (10b) gebildet wird.

8. System (1) nach Anspruch 6, wobei die Primärträgerflansche (14a-14d) sich außerhalb der Ebene erstrecken, die durch die flache Konfiguration des ersten Körperteils (10a) der Primärkomponente (10) gebildet wird.

9. System (1) nach einem der vorhergehenden Ansprüche, wobei die Sekundärkomponente (20) mehrere Sekundärflansche (22a-22d) umfasst, die sich von der optischen Achse (23) weg und in der Ebene erstrecken, die durch den optischen Körper der Sekundärkomponente (20) gebildet wird.

10. System (1) nach Anspruch 9, wobei jeder Sekundärflansch (22a-22d) während der Unterbringung in einer Flanschöffnung (12a-12d) beweglich ist, die in dem Umfang des zweiten Körperteils (10b) der Primärkomponente (10) bereitgestellt wird.

11. System (1) nach Anspruch 9 oder 10, wobei jeder Sekundärflansch (22a-22d) mit einer Öffnung (24a-24d) ausgestattet ist.

12. System (1) nach Anspruch 9, wobei jeder Sekundärflansch (22a-22d) mit einem abgerundeten Rand (25) ausgestattet ist, wobei der abgerundete Rand (25) so vorgesehen ist, dass er um den Außenumfang des ersten Primärkomponentenkörperteils (10a) herum eingehakt werden kann.

13. System (1) nach einem oder mehreren der vorhergehenden Ansprüche, wobei die Primärkomponente (10) einen Träger bildet und die Sekundärkomponente (20) eine Linse bildet.

14. System (1) nach einem oder mehreren der vorhergehenden Ansprüche, wobei die Primärkomponente (10) aus einem Material mit einer Kontrast erhöhenden Farbe relativ zu der optischen Sekundärkomponente (20) gefertigt ist.

## Revendications

1. Système de lentille intraoculaire (1, 1') pour implantation dans une capsule de lentille (106) d'un œil (100) de corps humain ou animal, ladite capsule de lentille (106) ayant un périmètre de capsulorhexis, ledit système de lentille intraoculaire (1, 1') comprenant:
i) un composant primaire intraoculaire (10) ayant un corps présentant une configuration plate; et
ii) un composant secondaire intraoculaire (20) ayant un corps optique avec un axe optique (23),
dans lequel le composant primaire (10) comporte une première partie de corps de composant primaire (10a) configurée pour s'adapter à l'intérieur de la capsule de lentille (106) et une deuxième partie de corps de composant primaire (10b) configurée pour être positionnée à l'extérieur de la capsule de lentille (106) et dans la chambre postérieure (109) de l'œil (100) et dans lequel la deuxième partie de corps de composant primaire (10b) est configurée pour recevoir de manière amovible le composant secondaire (20) dans la chambre postérieure (109) de l'œil (100) pour fournir une correction optique, de sorte que le composant secondaire (20) soit mobile par rapport au composant primaire (10).

2. Système (1) selon la revendication 1, dans lequel la deuxième partie de corps de composant primaire (10b) est configurée pour être positionnée au même niveau que la ligne de surface antérieure de la capsule de lentille (106) et dans la chambre postérieure (109) de l'œil (100).

3. Système (1) selon la revendication 1 ou 2, dans lequel le composant primaire (10) comporte un trou central s'étendant dans une direction antéro-postérieure, dans lequel le trou est dimensionné et configuré pour recevoir le composant secondaire (20) dans la deuxième partie de corps de composant primaire (10).

4. Système (1) selon l'une quelconque ou plusieurs des revendications 1 à 3, dans lequel le corps optique du composant secondaire (20) est logé dans le plan formé par la configuration plate de la deuxième partie de corps (10b) du composant primaire (10).

5. Système (1) selon l'une quelconque ou plusieurs des revendications 1 à 3, dans lequel le corps optique du composant secondaire (20) est positionné à l'extérieur du plan formé par la configuration plate de la deuxième partie de corps (10b) du composant primaire (10).

6. Système (1) selon l'une quelconque ou plusieurs des revendications précédentes, dans lequel le composant primaire (10) est muni de deux brides de support primaires (14a-14d) ou plus s'étendant à partir de la première partie de corps (10).

7. Système (1) selon la revendication 6, dans lequel lesdites brides de support primaires (14a-14d) s'étendent dans le plan formé par la configuration plate de la première partie de corps (10a) du composant primaire (10b).

8. Système (1) selon la revendication 6, dans lequel lesdites brides de support primaires (14a-14d) s'étendent à l'extérieur du plan formé par la configuration plate de la première partie de corps (10a) du composant primaire (10).

9. Système (1) selon l'une quelconque ou plusieurs des revendications précédentes, dans lequel le composant secondaire (20) comprend de multiples brides secondaires (22a-22d) s'étendant loin de l'axe optique (23) et dans le plan formé par le corps optique du composant secondaire (20).

10. Système (1) selon la revendication 9, dans lequel chaque bride secondaire (22a-22d) est logée mobile dans une ouverture de bride (12a-12d) prévue dans la circonférence de la deuxième partie de corps (10b) du composant primaire (10).

11. Système (1) selon la revendication 9 ou 10, dans lequel chaque bride secondaire (22a-22d) est munie d'une ouverture (24a-24d).

12. Système (1) selon la revendication 9, dans lequel chaque bride secondaire (22a-22d) est munie d'un bord arrondi (25), ledit bord arrondi (25) étant agencé pour s'accrocher autour de la circonférence extérieure de la première partie de corps de composant primaire (10a).

13. Système (1) selon l'une quelconque ou plusieurs des revendications précédentes, dans lequel le composant primaire (10) constitue un support et le composant secondaire (20) constitue une lentille.

14. Système (1) selon l'une quelconque ou plusieurs des revendications précédentes, dans lequel le composant primaire (10) est fabriqué à partir d'un matériau ayant une couleur améliorant le contraste par rapport au composant optique secondaire (20).
